# EUROPEAN PATENT APPLICATION

(11) **EP 4 317 442 A1**
(43) Date of publication of application: **07.02.2024**
(21) Application number: 21935325.7
(22) Date of filing: 25.11.2021
(51) Int. Cl.: C12N 15/75, C12N 9/06, C12N 9/10, C12N 9/04, C12N 9/16, C12P 7/18

(54) **RECOMBINANT MICROORGANISM FOR PRODUCING 2,3-BUTANEDIOL WITH REDUCED BY-PRODUCT PRODUCTION AND METHOD FOR PRODUCING 2,3-BUTANEDIOL BY USING SAME**

(30) Priority: 02.04.2021 KR 20210043483
(71) Applicant: GS Caltex Corporation, Seoul 06141 (KR)
(72) Inventor: SONG, Chan Woo, Seoul 06141 (KR); SONG, Hyo Hak, Seoul 06141 (KR); RATHNASINGH, Chelladurai, Seoul 06141 (KR); PARK, Jong Myoung, Seoul 06141 (KR)
(74) Representative: Zacco Sweden AB
(86) International application number: PCT/KR2021/017564
(87) International publication number: WO 2022/211212

(57) **Abstract**

The present invention relates to a recombinant microorganism for producing 2,3-butanediol, in which, in a microorganism having the 2,3-butanediol biosynthetic pathway, at least one selected from a group consisting of a pathway for converting glutamic acid into polyglutamic acid, a pathway for converting sucrose into levan, a pathway for converting pyruvate into lactate, and a pathway for converting glycerophosphate into glycerol is inhibited. The present invention also relates to a method for producing 2,3-butanediol by using the recombinant microorganism for producing 2,3-butanediol.

## Description

### FIELD

The present disclosure relates to a recombinant microorganism for producing 2,3-butanediol with reduced by-product production and a method for producing 2,3-butanediol using the same.

### DESCRIPTION OF RELATED ART

2,3-butanediol as an alcohol (CH₃CHOHCHOHCH₃) with four carbons and two hydroxy groups (-OH) is converted to 1,3-butadiene as a raw material for the synthetic rubber preparation process, and methyl ethyl ketone (MEK) which is used as a fuel additive and a solvent in a chemically-catalytic manner (Ji et al., Biotechnol. Adv., 29: 351, 2011). Furthermore, 2,3-butanediol is an industrially very important intermediate because it may be mixed with gasoline and the mixture as applied as an octane booster (Celinska et al., Biotechnol. Adv., 27: 715, 2009).

2,3-butanediol produced via microorganism is a chemical substance of great value in terms of economic feasibility and industrial applicability. While production of 2,3-butanediol via a chemical catalyst process is complex and requires high process costs, production of 2,3-butanediol via biological fermentation and separation and purification processes not only enables economical production, but also allows the produced 2,3-butanediol to be applicable as raw materials for various high value-added products such as food, medicine, agricultural products, and cosmetics. Historically, it has been verified that 2,3-butanediol may be produced via various microorganisms such as Klebsiella, Enterobacter, Serratia, Escherichia coli, and Bacillus. A genetic toolbox for strain improvement of Bacillus licheniformis among the various microorganisms is well set up. Due to its high-temperature culturing (thermophlic) characteristics of Bacillus licheniformis, the production of 2,3-butanediol via Bacillus licheniformis may prevent contamination during fermentation production, reduce a cooling cost, and enable simultaneous saccharification and fermentation. Above all, Bacillus licheniformis may be a microorganism with great industrial value due to its GRAS (generally recognized as safe) characteristics.

Research on the development of microorganisms related to 2,3-butanediol production has mainly been conduct so as to reduce by-products. Representatively, Ji et al. succeeded in partially suppressing the production of organic acids as by-products by exposing wild-type Klebsiella oxytoca strain to UV, which is of a type of physical/chemical mutagenesis method (Ji et al., Biotechnol. Lett., 30: 731, 2008). In addition, there is an attempt to improve 2,3-butanediol production by applying the ion beam scheme to Klebsiella pneumoniae strains to increase the consumption rate of biomass (FIG. Ma et al., Appl. Microbiol. Biotechnol., 82: 49, 2009). Furthermore, there is a research result showing that the 2,3-butanediol production ability is increased by suppressing the ldh and pflB paths as the main by-product production metabolic paths of wild-type Klebziella microorganism in a metabolically engineering manner.

On the contrary, regarding Bacillus licheniformis, the wild type Bacillus licheniformis has a strong tendency to be mucoid, and thus, major metabolic chemicals thereof are different from those of the Klebsiella microorganism group. Thus, a different approach is needed in microorganism development strategies.

Therefore, the present inventors have studied recombinant Bacillus licheniformis with suppressed by-product production.

### DISCLOSURE

### TECHNICAL PURPOSE

A purpose of the present disclosure is to provide a recombinant microorganism, preferably a recombinant Bacillus licheniformis, for producing 2,3-butanediol with suppressed by-product production.

### TECHNICAL SOLUTION

In order to achieve the above purpose, the present disclosure provides a microorganism with a 2,3-butanediol biosynthetic pathway,
wherein the microorganism includes a recombinant microorganism for producing 2,3-butanediol,
wherein at least one selected from a group consisting of a polyglutamate biosynthetic pathway, a levan biosynthetic pathway, a lactate biosynthetic pathway, and a glycerol biosynthetic pathway is inhibited.

Moreover, the present disclosure provides a microorganism with a 2,3-butanediol biosynthetic pathway,
wherein the microorganism includes a recombinant microorganism for producing 2,3-butanediol,
wherein at least one selected from a group consisting of a path for converting glutamate to polyglutamate, a path for converting sucrose to levan, a path for converting pyruvate to lactate, and a path for converting glycerophosphate to glycerol is inhibited.

Moreover, the present disclosure provides a method for producing 2,3-butanediol, the method comprising:
culturing the recombinant microorganism for producing 2,3-butanediol according to the present disclosure; and
recovering 2,3-butanediol from the culturing product.

### TECHNICAL EFFECT

The recombinant microorganism in accordance with the present disclosure has the suppressed by-product production and thus is suitable for 2,3-butanediol production.

### BRIEF DESCRIPTION OF THE DRAWING

FIG. 1 shows a biosynthetic pathway of 2,3-butanediol in a recombinant microorganism in accordance with the present disclosure, and a recombinant microorganism production strategy.
FIG. 2 shows a cell shape of a wild-type and viscous polymer-removing microorganism.
FIG. 3 shows an amount of extracellular polymer produced by each of a wild-type microorganism of Comparative Example 1 and a recombinant microorganism of each of Preparation Examples 1 and 2 when being cultured in each of a glucose-containing culture medium and a sucrose-containing culture medium.
FIG. 4 shows 2,3-butanediol production and yield of each of the wild-type microorganism of Comparative Example 1 and the recombinant microorganism of each of Preparation Examples 1 and 2 when being cultured in each of a glucose-containing culture medium and a sucrose-containing culture medium.
FIG. 5A shows polyglutamate, levan, and ¹³C NMR chemical shifts thereof. FIG. 5B shows a NMR analysis result of polyglutamate and levan in a culturing product in culturing of the wild-type microorganism of Comparative Example 1. FIG. 5C shows a NMR analysis result of polyglutamate and levan in a culturing product in culturing of the recombinant microorganism of Preparation Example 1. FIG. 5D shows a NMR analysis result of polyglutamate and levan in a culturing product in culturing of the recombinant microorganism of Preparation Example 2.
FIG. 6 shows an analysis result of lactate and glycerol productions of a recombinant microorganism of each of Preparation Examples 2 to 4.
FIG. 7 shows analysis results of the 2,3-butanediol production and yield of the recombinant microorganism of each of Preparation Examples 2 to 4.
FIG. 8 shows analysis results of 2,3-butanediol and major by-product productions of the recombinant microorganism of Preparation Example 4 when being fermented in a glucose culture medium.
FIG. 9 shows analysis results of 2,3-butanediol and major by-product productions of the recombinant microorganism of Preparation Example 4 when being fermented in a sucrose culture medium.

### DETAILED DESCRIPTIONS

The present disclosure provides a microorganism with a 2,3-butanediol biosynthetic pathway,
wherein the microorganism includes a recombinant microorganism for producing 2,3-butanediol,
wherein at least one selected from a group consisting of a polyglutamate biosynthetic pathway, a levan biosynthetic pathway, a lactate biosynthetic pathway, and a glycerol biosynthetic pathway is inhibited.

Moreover, the present disclosure provides a microorganism with a 2,3-butanediol biosynthetic pathway,
wherein the microorganism includes a recombinant microorganism for producing 2,3-butanediol,
wherein at least one selected from a group consisting of a path for converting glutamate to polyglutamate, a path for converting sucrose to levan, a path for converting pyruvate to lactate, and a path for converting glycerophosphate to glycerol is inhibited.

Moreover, the present disclosure provides a method for producing 2,3-butanediol, the method comprising:
culturing the recombinant microorganism for producing 2,3-butanediol according to the present disclosure; and
recovering 2,3-butanediol from the culturing product.

Hereinafter, the present disclosure is described in detail.

### Microorganism with 2,3-butanediol biosynthetic pathway

The microorganism in accordance with the present disclosure has a 2,3-butanediol biosynthetic pathway. The 2,3-butanediol biosynthetic pathway refers to a path in which 2,3-butanediol is synthesized from specific metabolites within a microorganism. The 2,3-butanediol biosynthetic pathway in accordance with the present disclosure may be a path in which 2,3-butanediol is synthesized directly or indirectly from pyruvate, alpha-acetolactate and/or acetoin. Preferably, the microorganism of the present disclosure may include a path in which 2,3-butanediol is synthesized from acetoin.

Furthermore, the microorganism in accordance with the present disclosure may be a microorganism having a 2,3-butanediol biosynthetic pathway in a wild-type manner, or a recombinant microorganism having the 2,3-butanediol biosynthetic pathway via a genetic recombination. Preferably, the microorganism is a microorganism that has the ability to produce 2,3-butanediol. The microorganism may be a microorganism of the Klebsiella genus, Bacillus genus, Serratia genus, or Enterobacter genus, preferably, Bacillus licheniformis (B. licheniformis), Bacillus subtilis (B. subtilis), Bacillus amyloliquefaciens (B. amyloliquefaciens), Bacillus polymyxa (P. polymyxa), etc., most preferably, Bacillus licheniformis (B. licheniformis). In this regard, using Bacillus licheniformis is advantageous for industrial-scale production of 2,3 butanediol.

### Polyglutamate biosynthetic pathway

In the recombinant microorganism in accordance with the present disclosure, a polyglutamate biosynthetic pathway may be inhibited. The polyglutamate biosynthetic pathway in accordance with the present disclosure refers to the path in which polyglutamate is synthesized from specific metabolites within a microorganism. The polyglutamate biosynthetic pathway may be a path in which polyglutamate is synthesized from glutamate, that is, a path for converting glutamate to polyglutamate. The path in which polyglutamate is synthesized from glutamate may be a path in which polyglutamate is synthesized from glutamate using polyglutamate synthase.

The polyglutamate synthase regulates the conversion of glutamate to polyglutamate. Inhibiting the polyglutamate synthase may allow the path for converting glutamate to polyglutamate to be inhibited. The inhibition of polyglutamate synthase may be achieved by inhibiting the expression of polyglutamate synthase, inhibiting the enzyme activity of polyglutamate synthase, etc. For example, those skilled in the art may select an appropriate scheme to inhibit the polyglutamate synthase from a group consisting of a scheme of deleting pgsBCAE as a gene encoding the polyglutamate synthase, a scheme of causing a mutation of the gene (mutation suppressing the expression of normal genes via variation, substitution or deletion of some bases thereof, or via introducing some bases thereto), and a scheme of regulating the gene expression during transcription or translation, etc. The pgsBCAE gene may be a base sequence with 80% or greater homology with SEQ ID NO: 1. Preferably, it may be a base sequence with 85% or greater homology with SEQ ID NO: 1, and more preferably, it may be a base sequence with 90% or greater homology with SEQ ID NO: 1. Even more preferably, it may be a base sequence with 95% or greater homology therewith, and most preferably, it may be a base sequence with 100% homology therewith.

### Levan biosynthetic pathway

In the recombinant microorganism of the present disclosure, the levan biosynthetic pathway may be inhibited. The levan biosynthetic pathway in accordance with the present disclosure refers to the path in which levan is synthesized from specific metabolites within a microorganism. The levan biosynthetic pathway may be a path in which levan is synthesized from sucrose, that is, a path for converting sucrose to levan. The path in which levan is synthesized from sucrose may be a path in which levan is synthesized from sucrose using levansucrase.

levansucrase regulates the conversion of sucrose to levan. Inhibiting levansucrase may allow the path for converting sucrose to levan to be inhibited. The inhibition of levansucrase may be achieved by inhibiting the expression of levansucrase, inhibiting the enzyme activity of levansucrase, etc. For example, those skilled in the art may select an appropriate scheme to inhibit the levansucrase from a group consisting of a scheme of deleting sacB as a gene encoding the levansucrase, a scheme of causing a mutation of the gene (mutation suppressing the expression of normal genes via variation, substitution or deletion of some bases thereof, or via introducing some bases thereto), and a scheme of regulating the gene expression during transcription or translation, etc. The sacB gene may be a base sequence with 80% or greater homology with SEQ ID NO: 9. Preferably, it may be a base sequence with 85% or greater homology with SEQ ID NO: 9, and more preferably, it may be a base sequence with 90% or greater homology with SEQ ID NO: 9. Even more preferably, it may be a base sequence with 95% or greater homology therewith, and most preferably, it may be a base sequence with 100% homology therewith.

### Lactate biosynthetic pathway

In the recombinant microorganism of the present disclosure, the lactate biosynthetic pathway may be inhibited. The lactate biosynthetic pathway in accordance with the present disclosure refers to the path in which lactate is synthesized from specific metabolites within a microorganism. The lactate biosynthetic pathway may be a path in which lactate is synthesized from pyruvate, that is, a path for converting pyruvate to lactate. The path in which lactate is synthesized from pyruvate may be a path in which lactate is synthesized from pyruvate using lactate dehydrogenase.

The lactate dehydrogenase regulates the conversion of pyruvate to lactate. Inhibiting the lactate dehydrogenase may allow the path for converting pyruvate to lactate to be inhibited. The inhibition of lactate dehydrogenase may be achieved by inhibiting the expression of lactate dehydrogenase, inhibiting the enzyme activity of lactate dehydrogenase, etc. For example, those skilled in the art may select an appropriate scheme to inhibit the lactate dehydrogenase from a group consisting of a scheme of deleting ldh as a gene encoding the lactate dehydrogenase, a scheme of causing a mutation of the gene (mutation suppressing the expression of normal genes via variation, substitution or deletion of some bases thereof, or via introducing some bases thereto), and a scheme of regulating the gene expression during transcription or translation, etc. The ldh gene may be a base sequence with 80% or greater homology with SEQ ID NO: 17. Preferably, it may be a base sequence with 85% or greater homology with SEQ ID NO: 17, and more preferably, it may be a base sequence with 90% or greater homology with SEQ ID NO: 17. Even more preferably, it may be a base sequence with 95% or greater homology therewith, and most preferably, it may be a base sequence with 100% homology therewith.

### Glycerol biosynthetic pathway

In the recombinant microorganism of the present disclosure, the glycerol biosynthetic pathway may be inhibited. The glycerol biosynthetic pathway in accordance with the present disclosure refers to the path in which glycerol is synthesized from specific metabolites within a microorganism. The glycerol biosynthetic pathway may be a path in which glycerol is synthesized from glycerophosphate, that is, a path for converting glycerophosphate into glycerol. The path for converting glycerophosphate to glycerol may be a path in which the conversion of glycerophosphate to glycerol is controlled using glycerophosphatase.

The glycerophosphatase regulates the conversion of glycerophosphate to glycerol. Inhibiting the glycerophosphatase may allow the path for converting glycerophosphate to glycerol to be inhibited. The inhibition of the glycerophosphatase may be achieved by inhibiting the expression of glycerophosphatase, inhibiting the enzyme activity of glycerophosphatase, etc. For example, those skilled in the art may select an appropriate scheme to inhibit the glycerophosphatase from a group consisting of a scheme of deleting dgp as a gene encoding the glycerophosphatase, a scheme of causing a mutation of the gene (mutation suppressing the expression of normal genes via variation, substitution or deletion of some bases thereof, or via introducing some bases thereto), and a scheme of regulating the gene expression during transcription or translation, etc. The dgp gene may be a base sequence with 80% or greater homology with SEQ ID NO: 25. Preferably, it may be a base sequence with 85% or greater homology with SEQ ID NO: 25, and more preferably, it may be a base sequence with 90% or greater homology with SEQ ID NO: 25. Even more preferably, it may be a base sequence with 95% or greater homology therewith, and most preferably, it may be a base sequence with 100% homology therewith.

### Recombinant microorganism for producing 2,3-butanediol

The recombinant microorganism for producing 2,3-butanediol in accordance with the present disclosure may be a recombinant microorganism for producing 2,3-butanediol which is characterized in that the path for converting glutamate to polyglutamate is inhibited, while the path for converting sucrose to levan, the path for converting pyruvate to lactate, and the path for converting glycerophosphate to glycerol are not inhibited.

The recombinant microorganism for producing 2,3-butanediol in accordance with the present disclosure may be a recombinant microorganism for producing 2,3-butanediol which is characterized in that the path for converting glutamate to polyglutamate and the path for converting sucrose to levan are inhibited, while the path for converting pyruvate to lactate, and the path for converting glycerophosphate to glycerol are not inhibited.

The recombinant microorganism for producing 2,3-butanediol in accordance with the present disclosure may be a recombinant microorganism for producing 2,3-butanediol which is characterized in that the path for converting glutamate to polyglutamate, the path for converting sucrose to levan, and the path for converting pyruvate to lactate are inhibited, while the path for converting glycerophosphate to glycerol is not inhibited.

The recombinant microorganism for producing 2,3-butanediol in accordance with the present disclosure may be a recombinant microorganism for producing 2,3-butanediol which is characterized in that the path for converting glutamate to polyglutamate, the path for converting sucrose to levan, the path for converting pyruvate to lactate, and the path for converting glycerophosphate to glycerol are inhibited.

The recombinant microorganism for producing 2,3-butanediol in accordance with the present disclosure may increase a productivity of 2,3-butanediol (an amount of 2,3-butanediol produced per unit time and unit volume), a production ability (an amount of 2,3-butanediol produced per unit volume, i.e., a concentration of 2,3-butanediol in a fermented product), and a yield (a production of 2,3-butanediol relative to an amount of a carbon source). In particular, the recombinant microorganism may increase a concentration of 2,3-butanediol in the fermented product during the fermentation, compared to the wild-type microorganism.

When culturing the recombinant microorganism in accordance with the present disclosure, the production of viscous polymers, lactate, or glycerol may be reduced, compared to the wild-type microorganism.

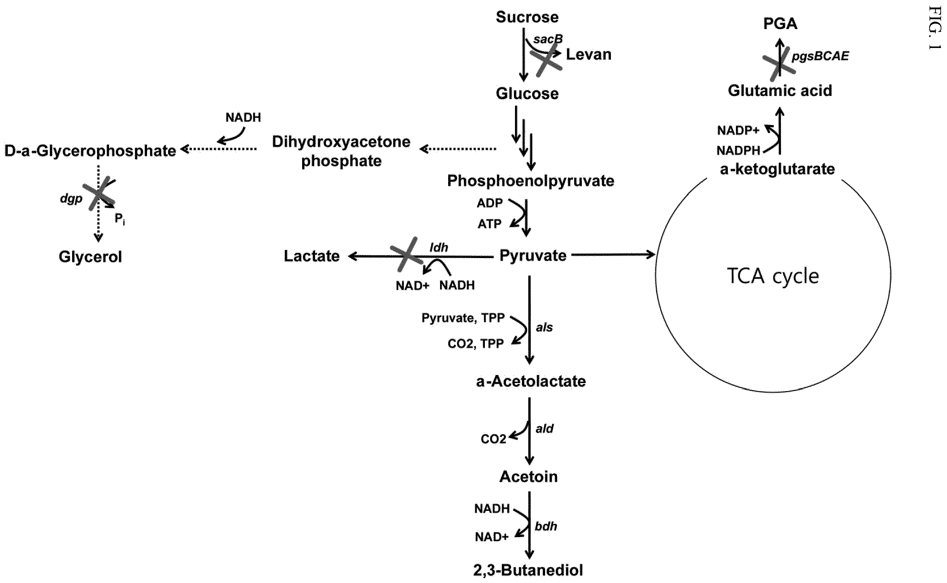

The biosynthetic pathway of the 2,3-butanediol in the recombinant microorganism in accordance with the present disclosure, and the recombinant microorganism production strategy are shown in FIG. 1.

### Step of culturing recombinant microorganism for producing 2,3-butanediol

The method for producing 2,3-butanediol in accordance with the present disclosure may include culturing the recombinant microorganism for producing 2,3-butanediol in accordance with the present disclosure. The culturing may be performed under an aerobic condition, preferably under a microaerobic condition. For example, the culturing may be performed while supplying oxygen, that is, air thereto during the culturing. In a specific example, this approach may be accomplished via stirring. However, the present disclosure is not limited thereto. The recombinant microorganism in accordance with the present disclosure may be cultured in a composite culture medium. It is obvious that the type of the composite culture medium is not particularly limited, and those skilled in the art may appropriately select and use the composite culture medium that is generally commercially available and used.

### Step of recovering 2,3-butanediol from culturing product

The 2,3-butanediol production method of the present disclosure may include the step of recovering 2,3-butanediol from the culturing product. The recovering step is not particularly limited, and may be performed in a conventional scheme of recovering a specific product from the culturing product obtained using the microorganisms.

### Production method of 2,3-butanediol

The present disclosure provides a method for producing 2,3-butanediol using the recombinant microorganism for producing 2,3-butanediol in accordance with the present disclosure.

### Examples

The advantages and features of the present disclosure, and a method to achieve the same will become clear with referring to examples as described in detail below. However, the present disclosure is not limited to the examples disclosed below and will be implemented in various different forms, but these examples are provided to only ensure that the present disclosure is complete and to fully inform those skilled in the art of the scope of the present disclosure. However, the scope of the present disclosure is only defined by the scope of the claims.

### <Materials and Methods>

Bacillus licheniformis GSC4071 (KCTC 14485BP) as a wild-type strain isolated from a soil sample near Daejeon city was prepared. In following experimental examples, viscous polymer is a general term of a solid substance as obtained by removing cells from the culturing product and performing precipitation thereon with ethanol. Specifically, the viscous polymer is a general term of several types of viscous polymers including polyglutamate and levan, and other viscous polymers.

A concentration (g/L), a yield (g/g), and a productivity (g/L/h) of 2,3-butanediol were calculated as follows:
- 2,3-butanediol concentration (g/L): an amount of 2,3-butanediol produced per unit volume (L)
- 2,3-butanediol yield (g/g): (2,3-butanediol production (g)/a carbon source amount (g)) × 100
- 2,3-butanediol productivity (g/L/h): an amount (g) of 2,3-butanediol produced per unit time (hour) and unit volume (L)

### <Experimental Example 1>

Recombinant strains were prepared using Bacillus licheniformis GSC4071.

### <1-1> Preparing recombinant B. licheniformis in which pgsBCAE, sacB, ldh, and dgp are deleted

### Preparation of DNA fragment including homologous region of target gene

To inactivate the target gene of Bacillus licheniformis, recombination mechanism of bacteria was used, and the homologous region of the gene to be removed was amplified via PCR. Afterwards, a corresponding DNA fragment containing the homologous region was delivered to the bacteria, and then, the target gene was removed using the recombination mechanism via a recombinase between the homologous region of the gene in the DNA fragment and a gene in a chromosome of Bacillus licheniformis.

First, in order to clone a homologous region of the polyglutamate synthase of Bacillus licheniformis, a homologous region 1 (SEQ ID NO: 2) of the pgsBCAE (SEQ ID NO: 1) as the target gene was subjected to PCR amplification using primers of SEQ ID NOs: 3 and 4. Moreover, a homologous region 2 (SEQ ID NO: 5) thereof was amplified via PCR using primers of SEQ ID NOs: 6 and 7. Afterwards, the homologous regions 1 and 2 were simultaneously amplified via PCR using both as a template. Thus, a DNA fragment (SEQ ID NO: 8) containing the homologous regions 1 and 2 had been completed.

Further, in order to clone a homologous region of levansucrase of Bacillus licheniformis, a homologous region 1 (SEQ ID NO: 10) of the target gene sacB (SEQ ID NO: 9) was amplified via PCR using primers of SEQ ID NOs: 11 and 12. Furthermore, a homologous region 2 (SEQ ID NO: 13) was amplified via PCR using primers of SEQ ID Nos: 14 and 15. Afterwards, the homologous regions 1 and 2 were simultaneously amplified via PCR using both as a template. Thus, a DNA fragment (SEQ ID NO: 16) containing the homologous regions 1 and 2 had been completed.

Furthermore, in order to clone a homologous region of lactate dehydrogenase of Bacillus licheniformis, a homologous region 1 (SEQ ID NO: 18) of the target gene ldh (SEQ ID NO: 17) was amplified via PCR using primers of SEQ ID NOs: 19 and 20. Furthermore, a homologous region 2 (SEQ ID NO: 21) was amplified via PCR using primers of SEQ ID NOs: 22 and 23. Afterwards, the homologous regions 1 and 2 were simultaneously amplified via PCR using both as a template. Thus, a DNA fragment (SEQ ID NO: 24) containing the homologous regions 1 and 2 had been completed.

Moreover, in order to clone a homologous region of glycerophosphatase of Bacillus licheniformis, a homologous region 1 (SEQ ID NO: 26) of the target gene dgp (SEQ ID NO: 25) was amplified via PCR using primers of SEQ ID NOs: 27 and 28. Furthermore, a homologous region 2 (SEQ ID NO: 29) was amplified via PCR using primers of SEQ ID NOs: 30 and 31. Afterwards, the homologous regions 1 and 2 were simultaneously amplified via PCR using both as a template. A DNA fragment (SEQ ID NO: 32) containing the homologous regions 1 and 2 had been completed. (Table 1)

**Table 1**

| SEQ ID NO | sequences |
|---|---|
| 1 | atgtgggtaatgctattagcctgtgtgatcgttgttgggatcggcatttatgaaaaaaggcgccaccagc aaaatatcgatgcgctgcctgtccgagtgaacatcaacggtatacgcggaaagtccacggtgacaa gattaacaacagggatattaatcgaagcaggctacaaaacagtaggaaaaacaaccgggacagac gcaaggatgatttattgggacacaccggaagagaagccgatcaaaagaaagccgcaagggccga atatcggagagcagaaggaggttatgaaagaaacggtggaaagaggggccaatgcgattgtcagtg agtgcatggccgttaatcctgattaccaaatcatctttcaggaagaattgcttcaggctaatatcggcgtg atcgtgaacgtgctggaggatcacatggatgtgatgggaccgactttggatgaaatcgcagaagcatt cacagcaaccattccttataatggacatttggttattactgatagtgagtataccgatttctttaagcaaatt gcaaaagaaaggaacacaaaagtcatcgtcgcagacaattctaaaataacagatgaatacctcaga cagtttgagtacatggtattccctgataatgcgtctcttgcgctcggtgtagctcaagcgttgggcattga cgaagaaaccgcctttaaaggcatgctgaatgcgccgcctgatccgggagccatgagaattctgcc gctgatgaacgccaagaatcccggacatttcgtcaacggttttgcggccaatgacgcagcttccacttt aaacatttggaagcgtgtaaaagaaataggctatcctacggatcagccgatcgtcattatgaactgcc gcgccgacagggtagacagaacacagcagtttgcggaagatgtccttccttatattgaagcaagtga acttgtgctgattggagaaacaacagagccgatcgtcaaagcatatgaagcaggcaaaattcctgcg gacaagctgtttgattttgagcacaaatcaacggaagaaatcatgttcatgctgaaaaacaagcttgag ggccgcgttatttacggagtcggaaatatccacggagcagcggagcctctcattgaaaaaatacaag attacaagattaagcagctcgttagctaggaggaagaaaacacaatgtttggatcagatttatatatcgc cctcattttaggagtcttactcagtttgatttttgcggagaaaacgggaattgtaccagccggcctcgtcg taccgggttatttgggacttgtcttcaatcagccgattttcatgctgctcgttctttttgtcagtttgctgacgt atgtcatcgtgaaattcggactttccaaaattatgattctatacggacgcagaaaattcgcagcaatgct gattacgggaattcttttgaaaatcggttttgattttatatatccggtgatgccgtttgagattgccgaattca ggggaatcggaatcatcgtgccggggctgatcgccaataccattcaaagacagggattaacgattac gcttggaagtacgcttttattgagcggagcaacattcgtcattatgtatgcttactatctaatctaaagttag gtgtgtcaaatcgatgaaaaaacaactgaactttcaggaaaaactgctgaagttgacgaagcaggag aaaaagaaaacaaacaagcacgtctttatcgtattgcccgttattttctgtttaatgtttgtctttacttgggt cggaagcgccaaaactccttcgcaaatggacaaaaaagaagatgccaagcttacagctacttttgttg gcgatatcatgatgggaagaaacgtagaaaaagtgacaaacttgcacggttcggaaagtgtcttcaa aaatgtgaagccgtactttaatgtgtcagattttatcacaggaaactttgaaaaccctgtaaccaatgca aaggactatcaagaggcagaaaagaacatccatctgcaaacgaatcaagaatcagtcgaaacattg aaaaagctgaacttcagcgtactgaattttgccaacaaccatgcgatggactacggggaagacggttt gaaggatacgctcaataaattttcaaatgagaatctggagcttgtcggagcaggaaataatcttgaaga cgcgaaacagcacgtatcctatcagaatgtgaacggcgtaaaaattgcaacgctcggttttacagac gtctacacaaagaactttacagccaaaaagaacagaggcggagtgctgccgctcagtccgaaaatc tttattccaatgattgcggaagcatcgaaaaaagcggatcttgtccttgtccatgtgcactggggacaag aatatgacaatgaaccgaacgacagacagaaggatctggccaaggcgattgcagatgccggagca gatgtcatcatcggcgctcatccccatgttctcgaaccgatcgaagtgtataacggtactgtgattttcta cagcctcggcaactttgtatttgatcagggctggtcaagaacacgggacagcgcgcttgtacaatacc atttaatgaatgacggcaaagggcgttttgaggtaacgcctctcaacattcgcgaagcaacgccgac gcctttaggcaagagcgacttcttaaaacgaaaagcgatcttccgtcaattgacaaaaggaacaaac ctcgactggaaagaagagaacggaaaattaacgtttgaagtcgatcatgcggacaagctgaaaaaat aataaaaacggagtggtgaacaaatgaaatttgtcagagccatttggccgttcgtaggtttagttttgatc |
| | attgcattcatgtctgctttcaagtattctgatgaattgtcaaacgatgaaaaagctaaaatctctacggag atccaaaaagtgaatcagcaggatcaaacaacagaaaacaagcaataa |
| 2 | aagccttctcctctctatttgtgtaaccgtttacataaagattatcacgatccatttaaaatcacaataaaaa aatggatgaacactgaaattttagaccgggctgggatatcgagacatatataggggcgtttaatggcga atacagtaacaatgagaatagtaagaaaaattaaaaatgttaaagtttgatgaattatcattgaaaaaaat taatggctttttaaatcctaggattttaacctaaaatctgaagaaataaggtggatcgaacgactcacaaa atatttggatttgtcaatgaatcccgctttatgctaaaagagattttcattttttgatagatggtctgattgtcat aggacggatttgttttgaagagggaacattggtgactttttaacctgttcgaaaagagcgaaaatactaa aagaaaagagagatcccggctgacagcccatttaaaggggattgcggacgggggaaaaaagaga tcctgaatccatccttcaacctttcatctgaaatagggagaaaagtacaaaaatcataatgtcgaattttg aaagcgcatacttaaaacgctgacaaaaatctgataggaattaagaactttcgatttccaaaaatatca ataaaaagataggcattaatgactcgggcgaggtgatctttgtcacggaaaatttcgtcgtcttctgttac ataatgccgattgtgatttcatagtgaacctatatactgatgaatgaatttacatatcagattccaagaagg agatgtagacaaaca |
| 3 | agacagggccaacgaggccaagccttctcctctctatttgtgtaac |
| 4 | cggcactctcttttgcactgtgtttgtctacatctccttc |
| 5 | cagtgcaaaagagagtgccgccggcgctctcttttttgtgtgctgatcccggttataaaagacctgtga aaagcgaccggtttgaaagggaaacacgacaattttcttaaccggtcagtgtataaagttttatagaaa atcaggaggatatatacatggttttggggttcatgtttattgtattcttttgaagggaataaaaactgacaaa tttcgactgaagcaaaatttgaaaatgcatcaccttaccaattcgggatgggaaccgcacctcatgttc atgacctctttagaatatttcccttcatctttttaatctgcgcttaggtgaaaaagctgatcatgctgtgctga gcgtttcttctcgctatgacgctgctgtacatgcaaaaaaagtcctttaaatatcccagttgaatgacgat gaaagaggaaagaagaggaggaacagatcaattgataaaaaaagcggcaaacaaaaagttggtttt gttttgtggaattgcggtgctttggatgtctttatttttaacgaatcataatgatgtacgcgccgatacgatcg gcgagaaaatagcggaaactgccagacagcttgagggtgcgaaatacagctacggcggagagaa gccgaaaacggggtttgactcgtcaggctttgtgcaatatgtgtttcaatcgctcgatattacgcttccga gaacggtaaaggaacaatcgactcttgggagcagt |
| 6 | gaaggagatgtagacaaacacagtgcaaaagagagtgccg |
| 7 | agacagggccttattggccactgctcccaagagtcgattgttc |
| 8 | aagccttctcctctctatttgtgtaaccgtttacataaagattatcacgatccatttaaaatcacaataaaaa aatggatgaacactgaaattttagaccgggctgggatatcgagacatatataggggcgtttaatggcga atacagtaacaatgagaatagtaagaaaaattaaaaatgttaaagtttgatgaattatcattgaaaaaaat taatggctttttaaatcctaggattttaacctaaaatctgaagaaataaggtggatcgaacgactcacaaa atatttggatttgtcaatgaatcccgctttatgctaaaagagattttcattttttgatagatggtctgattgtcat aggacggatttgttttgaagagggaacattggtgactttttaacctgttcgaaaagagcgaaaatactaa aagaaaagagagatcccggctgacagcccatttaaaggggattgcggacgggggaaaaaagaga tcctgaatccatccttcaacctttcatctgaaatagggagaaaagtacaaaaatcataatgtcgaattttg aaagcgcatacttaaaacgctgacaaaaatctgataggaattaagaactttcgatttccaaaaatatca ataaaaagataggcattaatgactcgggcgaggtgatctttgtcacggaaaatttcgtcgtcttctgttac ataatgccgattgtgatttcatagtgaacctatatactgatgaatgaatttacatatcagattccaagaagg agatgtagacaaacacagtgcaaaagagagtgccgccggcgctctcttttttgtgtgctgatcccggtt ataaaagacctgtgaaaagcgaccggtttgaaagggaaacacgacaattttcttaaccggtcagtgta taaagttttatagaaaatcaggaggatatatacatggttttggggttcatgtttattgtattcttttgaagggaa taaaaactgacaaatttcgactgaagcaaaatttgaaaatgcatcaccttaccaattcgggatgggaac |
| | cgcacctcatgttcatgacctctttagaatatttcccttcatctttttaatctgcgcttaggtgaaaaagctga tcatgctgtgctgagcgtttcttctcgctatgacgctgctgtacatgcaaaaaaagtcctttaaatatccca gttgaatgacgatgaaagaggaaagaagaggaggaacagatcaattgataaaaaaagcggcaaac aaaaagttggttttgttttgtggaattgcggtgctttggatgtctttatttttaacgaatcataatgatgtacgc gccgatacgatcggcgagaaaatagcggaaactgccagacagcttgagggtgcgaaatacagcta cggcggagagaagccgaaaacggggtttgactcgtcaggctttgtgcaatatgtgtttcaatcgctcg atattacgcttccgagaacggtaaaggaacaatcgactcttgggagcagt |
| 9 | atgaacatcaaaaacattgctaaaaaagcgtcagccttaaccgttgctgcggcactgctggccggag gtgcgccgcaaacctttgcaaaagaaacgcaggattacaagaaaagctacggattttctcatatcaca agacatgacatgctgaaaattcccgagcagcaaaagagcgaacaatttaaagttcctcaattcgatcc gaaaacaatcaaaaacatcccttctgcaaaagggtataacaaaaatggagagctgatcgatttagac gtatgggacagctggccgctgcaaaatgccgacgggacggttgctacataccacggctacaatcttg ttttcgcgctggcgggcgatccgaaagacgtcgatgacacatccatctatttgttctatcaaaagaaag gcgaaacttctatcgacagctggaaaaacgccggcagagtgtttaaagacagcgacaaatttgttcc agacgatccgtacctcaaacatcaaacacaggaatggtcaggttctgccacgctgacaaaagacgg aaaagtccgactgttttacacagctttttccggcacgcaatacggcaagcagacgctgacaacagctc aggtcaatttctctcagccggattcggacacgctcaaaattgacggtgtagaagatcataaatcggtctt tgacggcgccgacggcacggtataccaaaacgttcagcaattcattgacgaaggaaactacagctc cggcgacaaccatacgatgagagacccgcattatgtggaagaccgcggccataaatatctcgtattt gaagccaatacgggaacaaaaaccggctaccaaggagaagactccctattcaacagagcctacta cgggggcagcaagaagttctttaaagaagaaagcagcaagctgctgcaaggtgcgaacaaaaaga acgcttcgctggctaacggcgctctcggaatcatcgaattaaataacgattatacactgaaaaaagtca tgaagcctttgatcgcctccaatacggtgacagatgaaatcgaacgggccaacctcttcaaaatgaat ggaaaatggtatctgttcacagattcaagaggatcaaaaatgacaattgacggcatcggttcaaaaga catttatatgctcggctatgtatcaggttcattaaccggaccattcaagcctttaaacaaatccggacttgt tttgcatatggaccaggattacaatgacatcacgtttacttattcacactttgccgtaccgcagaaaaaa ggcgacgaagtcgtcattacaagctacatcacaaacagagggatttcgaacgagcatcacgccacg tttgcaccaagctttttgctgaagatcaaaggatcaaaaacatccgttgtcaaaaacagcatccttgaac agggacaactaacggtaaacaaataa |
| 10 | gattacctgcacgtatcactggttgatttccggtccttgccgcgcaggggtgcagacagatcccgcgc aaggatggaactgatcaaagaagccgtccaagaccgtctcccactgatcggagccgggcaagtac acacaccggaagacgcccttcaagcgctggaaacaggagccgatctcatcggaatcgggcgggg tctcctgatggatccggagtggctacaaaaagttcgagccggaaaagaggacgatattcaaacaacc ctgtcaagacatgatcagcggaagctcgcaattccagatgccttttgggaaagtataccgatgtatattg atttcagcgattgacgattcccgcttatacagactatagattcatataaaaaaggtgtctttccgcttaaaa tcggtgtcatttgcataaaaatgtataggaaaagaggaacttagaccagttgagtcccagagatgagta tattagaatgatggtaattcaatatcgtcgggattgttactgtctaagcaggcaagacctaaaatgtgtga agggcgaaatctatcttttgcctatatgaacttgcacattttaggtcttttttctgttcttcaggacaatgccg cagttaggagggatgattgggaacgttcatgttgtcagaagcaatctattacatattgaaaaagggagg aaatattg |
| 11 | agacagggccaacgaggccgctagcgattacctgcacgtatcactg |
| 12 | tttccttcgttcgttcaatatttcctccctttttcaatatg |
| 13 | aacgaacgaaggaaaatgccggtgaacatcggcattttctttcgtatgtcatcttagaataaagcgggg aattttcttgaagaaggcagtatataagcggatcagcattttaactgtttgtcttggaatcttcatcatgatat |
| | ttctttcagtccgggaaacagaaaaaaagggagaatctccggatacaccggaatatcgtgcagctttc catttgacaacccctgacaaatggaaaaacgaccctcaaaaacccgtctatttcaatggagaatatca ttactactacctctataaccgagactatccggacggcaatggaacagagtggcgccacgcagtatca gatgatctcgtccactggcaggataaaggggttgccattccgaaatatacgaataaaaatggcgatcc ctggtccggttctgtcgttgtcgactcaaaaaacacagccggcttcggcaaagggacgatcgtggcg gttatgacgcagccgtcggccaatgacgggaaggaagagcaatttttatggtacagccaaaatggag gaaaaacatttaaaccttacggtgaagagccggtcttgcctaatccgggcaccgtcgatttcagagat ccgaaagtgatctgggatgaacaggatg |
| 14 | agggaggaaatattgaacgaacgaaggaaaatgcc |
| 15 | agacagggccttattggcccttaagcatcctgttcatcccagatc |
| 16 | gattacctgcacgtatcactggttgatttccggtccttgccgcgcaggggtgcagacagatcccgcgc aaggatggaactgatcaaagaagccgtccaagaccgtctcccactgatcggagccgggcaagtac acacaccggaagacgcccttcaagcgctggaaacaggagccgatctcatcggaatcgggcgggg tctcctgatggatccggagtggctacaaaaagttcgagccggaaaagaggacgatattcaaacaacc ctgtcaagacatgatcagcggaagctcgcaattccagatgccttttgggaaagtataccgatgtatattg atttcagcgattgacgattcccgcttatacagactatagattcatataaaaaaggtgtctttccgcttaaaa tcggtgtcatttgcataaaaatgtataggaaaagaggaacttagaccagttgagtcccagagatgagta tattagaatgatggtaattcaatatcgtcgggattgttactgtctaagcaggcaagacctaaaatgtgtga agggcgaaatctatcttttgcctatatgaacttgcacattttaggtcttttttctgttcttcaggacaatgccg cagttaggagggatgattgggaacgttcatgttgtcagaagcaatctattacatattgaaaaagggagg aaatattgaacgaacgaaggaaaatgccggtgaacatcggcattttctttcgtatgtcatcttagaataa agcggggaattttcttgaagaaggcagtatataagcggatcagcattttaactgtttgtcttggaatcttca tcatgatatttctttcagtccgggaaacagaaaaaaagggagaatctccggatacaccggaatatcgt gcagctttccatttgacaacccctgacaaatggaaaaacgaccctcaaaaacccgtctatttcaatgg agaatatcattactactacctctataaccgagactatccggacggcaatggaacagagtggcgccac gcagtatcagatgatctcgtccactggcaggataaaggggttgccattccgaaatatacgaataaaaa tggcgatccctggtccggttctgtcgttgtcgactcaaaaaacacagccggcttcggcaaagggacg atcgtggcggttatgacgcagccgtcggccaatgacgggaaggaagagcaatttttatggtacagcc aaaatggaggaaaaacatttaaaccttacggtgaagagccggtcttgcctaatccgggcaccgtcga tttcagagatccgaaagtgatctgggatgaacaggatg |
| 17 | atgagaaaccaaaaagtcaatcgtacagttttgatcggtgcgggttttgttggaagcagctatgcattta cgttaattaaccaaggtatcacagacgaacttgtgatcattgatttaaataaagacaaagcaatgggcg atgttatggatctcaaccacggtaaagcatttgctccgcatcctgtgaaaacatggtacggcacatatg atgactgcaaagaagctgatattgtctgcgtatgcgccggagcgaaccaaaagccgggtgaaaccc gccttgatctagtcgaaaagaacttgaaaatttttaaaggaatcatcggagaagtcatggccagcggttt tgacggcatcttcctcgtcgctacaaaccctgtcgacatcttgacttatgcgacttggaaattcagcggc cttccgaaagaacgcgtcatcggaagcggcacaacgcttgacactgcccgtttccgctacttgctca gcgagtatttcggtgtcgctgcccacaatgcgcacggctatatcattggtgaacacggagatacggag ctgcctgtctggagccatgcgaatatcggcggagttcccgtcagcgacttgctgaaaagaaatgaga aatacaaagcggaagatctagatgaactgtttgacaatgtaaaaaacgccgcttaccacattattgaga aaaaaggcgcgacatactacggagttgccatgagcctcgcacgtattacgaaagcgatttatcgaaa tgaagaagcaattttgaccgtgagcgctcatcttgatggtgaatttggagaaaatgatgtttatatcggag ttccggcggttgtcggccgatgcggagcgagggaaattgttgagctcgatctgaacgaaaaggaaa aacaacaattcaagcatagcgcaggtgtgttaaaagcaattctaaaaccgcattttgaactgcaggcgt |
| | aa |
| 18 | cataacaacggaatcatcgtcaccagcattccgccaacagccgtctttttcggtccgaaacggtcggt taaaatgccgagcgcaatccggaaaaacgatccggccaaagtaggtatggcgacaatcaatccga cctgtgaaggcgataaatcaaaatcagccgtaacataaacaccaagcgcccccagcaacacccaa atcataaagctgacatcgaaatataaaaaagatgacaacaaggaaggcgcgtgtccgctcttcctca aatccgccaatttcaattgcaacactccccatccgatagattctagaatttatcatgacaaaatcgaata gggttggcagtatttttgtaagctttactaacaaactaaaagattgaatatttaaaatgttagtttggacagc actatagcaggcgataattgaattatgctgaagaatagtggtgttatgagcgctgaaaatgggaaacctt atattgatataggcccgatgcacttgcagataaaaccgtcttttgcaacgcgtttatcaatttttctttagac ataaggcatatttttaccagaaaatgtacaaatttaaggaagaaatacgtggagacacaaaatagacat aaataaaaatgtatatcacttgaaaaagtatgtgaagtattgcacaatatgaatgtgaagaatttcacaaa cagcttagtttaaaacaaacggcaaaggaatgatgagtc |
| 19 | agacagggccaacgaggcccataacaacggaatcatcgtcacc |
| 20 | cttcatggtgttcaggactcatcattcctttgccgtttg |
| 21 | ctgaacaccatgaagatactaacatcatgaagattgcgagatgatgaaaatgtggcagcagatatacg atccgtttggaaatctgtttatcagcgcttttgccgccatactgccgattcttttctttcttcttgcactgaccg tgttgaagatgaaaggaatcattgcgtcatttttgacgctggcggtcagctttattgtagcggtgttcttcttt cacatgccggctgcgaaagctgtctccgccgttttacttggaattgctaacggcctttggccgatcgga tatattgtcctgatggctgtatggctttacaaaatttctgtcaaaaccggaaagtttaaagtgatcagagcg agcattgccggaatttcgtccgaccagcggcttcagctattgctgatcgggttcagttttaacgcctttttg gaaggtgcggcaggcttcggtgtgccgattgcgatcagcgccgttcttcttgtcgagcttggttttaaac ctttgaaagcggcatccctatgtttaatagcgaatgcggcatccggagcctttggagccatcggaattc cggttatcacaggagcgcagatgggaggcttaacaccggttgagctttccagaacgctcgctttgatg ctgccggtcatcgctttctttgtcccgttcctgctcgttttcatattggacgggtttaaagg |
| 22 | aaggaatgatgagtcctgaacaccatgaagatactaacatcatg |
| 23 | agacagggccttattggcccctttaaacccgtccaatatgaaaac |
| 24 | cataacaacggaatcatcgtcaccagcattccgccaacagccgtctttttcggtccgaaacggtcggt taaaatgccgagcgcaatccggaaaaacgatccggccaaagtaggtatggcgacaatcaatccga cctgtgaaggcgataaatcaaaatcagccgtaacataaacaccaagcgcccccagcaacacccaa atcataaagctgacatcgaaatataaaaaagatgacaacaaggaaggcgcgtgtccgctcttcctca aatccgccaatttcaattgcaacactccccatccgatagattctagaatttatcatgacaaaatcgaata gggttggcagtatttttgtaagctttactaacaaactaaaagattgaatatttaaaatgttagtttggacagc actatagcaggcgataattgaattatgctgaagaatagtggtgttatgagcgctgaaaatgggaaacctt atattgatataggcccgatgcacttgcagataaaaccgtcttttgcaacgcgtttatcaatttttctttagac ataaggcatatttttaccagaaaatgtacaaatttaaggaagaaatacgtggagacacaaaatagacat aaataaaaatgtatatcacttgaaaaagtatgtgaagtattgcacaatatgaatgtgaagaatttcacaaa cagcttagtttaaaacaaacggcaaaggaatgatgagtcctgaacaccatgaagatactaacatcatg aagattgcgagatgatgaaaatgtggcagcagatatacgatccgtttggaaatctgtttatcagcgctttt gccgccatactgccgattcttttctttcttcttgcactgaccgtgttgaagatgaaaggaatcattgcgtca tttttgacgctggcggtcagctttattgtagcggtgttcttctttcacatgccggctgcgaaagctgtctcc gccgttttacttggaattgctaacggcctttggccgatcggatatattgtcctgatggctgtatggctttaca aaatttctgtcaaaaccggaaagtttaaagtgatcagagcgagcattgccggaatttcgtccgaccag cggcttcagctattgctgatcgggttcagttttaacgcctttttggaaggtgcggcaggcttcggtgtgcc gattgcgatcagcgccgttcttcttgtcgagcttggttttaaacctttgaaagcggcatccctatgtttaata |
| | gcgaatgcggcatccggagcctttggagccatcggaattccggttatcacaggagcgcagatggga ggcttaacaccggttgagctttccagaacgctcgctttgatgctgccggtcatcgctttctttgtcccgttc ctgctcgttttcatattggacgggtttaaagg |
| 25 | atgaagaaatgggcatttgtatcagactttgatgggacgatttccaaacaggatttttactggatggtcatt gataaatattttcccgaaggccgtgaattgttcaagaagtggaagtccggagaattaaaagatatcgaa tttttgggaaccgtttttgcttcgattaatcaaagcgaacaaaaaatcattgatgacatccattccataccg attgatgaatatgtgcctgacttcattcagcatgtccagaaaagcggcggcgacttctacattttaagcg ccggcacggattattacattcattatattttaaagaaatacgggattacagacgttgaggtctattcaaata aaggcttttttaaagaagacaatgttcatatggacattgatgagaatcattggcattactctgagcgctatg ggattgataaatcaaaggtcattcaaaagctgaaagaagagtatgagacggtttattttgcaggcgaca gtgaaccggattcgcacccggctaaatttgcggatgttacgtttgcaaaggatgcgctccaggatttgc tgcgtcagcagggggtgccatttgtagccgttgaaacatttgaagacattgaacaatatcttaaagaaa aagggcggatcgtctaa |
| 26 | cctaagtgataatgattcgcattatcaatgtaaatatagcagatcaatccctctctcgtctatcactttttttg ctaagtatgattagtgtggttttggttaatttgttactataaacagaaaaaggactgatgagagatggcag aaacgaacccggtgatcaaaaaattgcagttcaaggataatggacagcctgtactgattgtgaatccg cctgaggcatacagcggcgtcatcgctgaatttcaaggccctgttcatcatcacgccgaatcggacc aatatgattttgttcaggtattcgcagcgaccaacgcaaagctgcaggctttagccaaggaggctgaa cgtcatttggcggaagacggtctgttttggctttgctacccgaaaaaatcatcgaaagtctacaaaggat cggactgcagccgcgatacagtagccggactgcttgcagaccagggctatgaaccggttcgccaa attgcaattgatgaagactggtctgcgctcagatttcgcaaggcggaaaacatcaaaacgatgaagc ggaaatttgccgtaacagagaaaggtaagcagcgtacagatcaagaatgattatatacgcctcctttc agggggacgaatattcagcttgtgaacgaaagtacacaaaaaagaaataatattatgttcaatgattca caaactttttgtataatgaatctaatcttttaaaataccatagaaaagaggtatgcagc |
| 27 | agacagggccaacgaggcccctaagtgataagtattcgcattatc |
| 28 | cggcattagacgatcgctgcatacctcttttctatgg |
| 29 | gatcgtctaatgccgcgccaggttaccagcatagccatccccgcggttttggacattagtgagggcgt gcttggccggttggatgagatattgagaaaacatcagtttgacaaggcgatcatgttttttgacgatttttc ctatcagcagtatgagcattcactgaaaaccgcatttcaacatgttaaagttgaagccgttctgctgccg tccaatctggacatacaggagcttttgaagcgggcattttcgatcgggaattgcgatgtcattattgccat gggcggcgggtatgttgtggactgcggaaaatacatcgccttttccaagcggaccccgttcatcagc attccgacgtcggcatccaatgacgggtttgcgagcagcaattgctcgcttcaggtcgaaggaaaaa aaacaaccgttccggcaagggttccctacggaatcatagctgatttgagcattattcaaaaagcgccg gattgctttattttggcggggatcggagatttgatgtccaatattacggccctttatgattgggagtttgagg agcggcacggggtcggcgatgtaaatgcgtttgcgtcgatgctcagcaagaaagcggtcaacagctt tgtccgtacgccgatgcaggatattaaacagccgatcttcttaaaagagcttgtcagttctttgacaatgg gcggcatcgctacggaaatcagcggaaac |
| 30 | aaagaggtatgcagcgatcgtctaatgccgcgc |
| 31 | agacagggccttattggccgtcaaagaactgacaagctcttttaag |
| 32 | cctaagtgataatgattcgcattatcaatgtaaatatagcagatcaatccctctctcgtctatcactttttttg ctaagtatgattagtgtggttttggttaatttgttactataaacagaaaaaggactgatgagagatggcag aaacgaacccggtgatcaaaaaattgcagttcaaggataatggacagcctgtactgattgtgaatccg cctgaggcatacagcggcgtcatcgctgaatttcaaggccctgttcatcatcacgccgaatcggacc aatatgattttgttcaggtattcgcagcgaccaacgcaaagctgcaggctttagccaaggaggctgaa |
| | cgtcatttggcggaagacggtctgttttggctttgctacccgaaaaaatcatcgaaagtctacaaaggat cggactgcagccgcgatacagtagccggactgcttgcagaccagggctatgaaccggttcgccaa attgcaattgatgaagactggtctgcgctcagatttcgcaaggcggaaaacatcaaaacgatgaagc ggaaatttgccgtaacagagaaaggtaagcagcgtacagatcaagaatgattatatacgcctcctttc agggggacgaatattcagcttgtgaacgaaagtacacaaaaaagaaataatattatgttcaatgattca caaactttttgtataatgaatctaatcttttaaaataccatagaaaagaggtatgcagcgatcgtctaatgc cgcgccaggttaccagcatagccatccccgcggttttggacattagtgagggcgtgcttggccggttg gatgagatattgagaaaacatcagtttgacaaggcgatcatgttttttgacgatttttcctatcagcagtat gagcattcactgaaaaccgcatttcaacatgttaaagttgaagccgttctgctgccgtccaatctggac atacaggagcttttgaagcgggcattttcgatcgggaattgcgatgtcattattgccatgggcggcgggt atgttgtggactgcggaaaatacatcgccttttccaagcggaccccgttcatcagcattccgacgtcgg catccaatgacgggtttgcgagcagcaattgctcgcttcaggtcgaaggaaaaaaaacaaccgttcc ggcaagggttccctacggaatcatagctgatttgagcattattcaaaaagcgccggattgctttattttgg cggggatcggagatttgatgtccaatattacggccctttatgattgggagtttgaggagcggcacgggg tcggcgatgtaaatgcgtttgcgtcgatgctcagcaagaaagcggtcaacagctttgtccgtacgccg atgcaggatattaaacagccgatcttcttaaaagagcttgtcagttctttgacaatgggcggcatcgcta cggaaatcagcggaaac |

### <1-2> Preparing recombinant B. licheniformis in which pgsBCAE, sacB, ldh, and dgp are deleted

The prepared DNA fragments were transferred to Bacillus licheniformis GSC4071 using electroporation (25 uF, 200 Ω, 2.5 kV/cm). The target genes were removed using the recombinant mechanism of the microorganism.

The recombinant Bacillus licheniformis from which the pgsBCAE gene was removed was prepared by transferring the DNA fragment (SEQ ID NO: 8) containing the homologous region of the pgsBCAE gene to Bacillus licheniformis GSC4071 (wild type). The recombinant microorganism as prepared in this way in which the pgsBCAE was deleted was used as a strain (B. licheniformis 4071 ΔpgsBCAE) of Preparation Example 1.

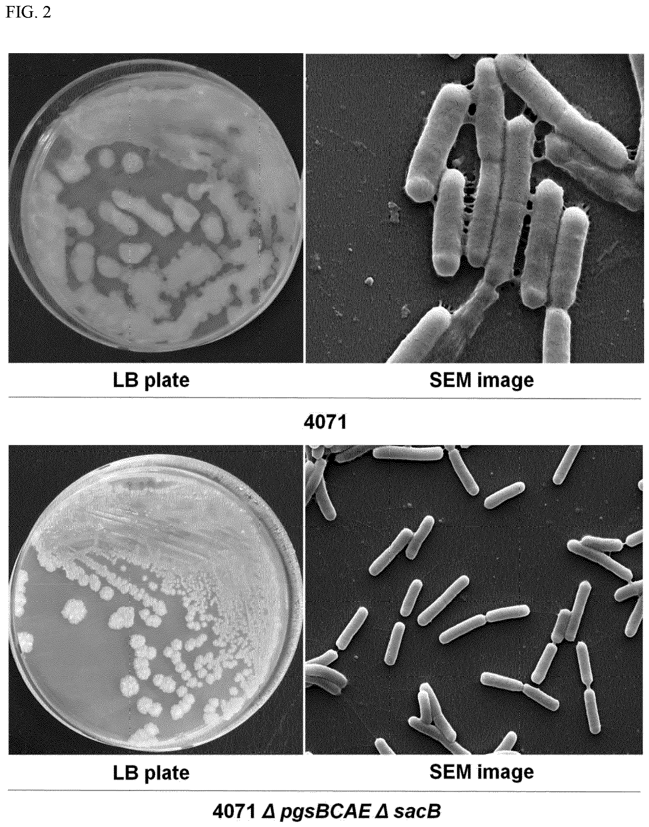

Further, as described above, the pgsBCAE gene was removed from Bacillus licheniformis GSC4071 (wild type), and then, the DNA fragment (SEQ ID NO: 16) containing the homologous region of the sacB gene was delivered thereto to prepare Bacillus licheniformis in which the sacB gene in addition to the pgsBCAE gene were deleted. The recombinant microorganism as prepared in this way in which pgsBCAE and sacB were deleted was used as a strain (B. licheniformis 4071 ΔpgsBCAE ΔsacB) of Preparation Example 2. A cell shape thereof is shown in a lower panel of FIG. 2.

Further, as described above, the pgsBCAE and sacB genes were removed from Bacillus licheniformis GSC4071 (wild type), and then, the DNA fragment (SEQ ID NO: 24) containing the homologous region of the ldh gene was delivered thereto to prepare Bacillus licheniformis in which the ldh gene in addition to the pgsBCAE and sacB genes were deleted. The recombinant microorganism as prepared in this way in which pgsBCAE, sacB, and ldh were deleted was used as a strain (B. licheniformis 4071 ΔpgsBCAE ΔsacB Δldh) of Preparation Example 3.

Further, as described above, the pgsBCAE, sacB, and ldh genes were removed from Bacillus licheniformis GSC4071 (wild type), and then the DNA fragment (SEQ ID NO: 32) containing the homologous region of the dgp gene was transferred thereto to prepare Bacillus licheniformis in which the dgp gene in addition to the pgsBCAE, sacB, and ldh genes were removed. The recombinant microorganism prepared in this way in which pgsBCAE, sacB, ldh, and dgp were deleted was used as a strain (B. licheniformis 4071 ΔpgsBCAE ΔsacB Δldh Δdgp) of Preparation Example 4.

In the following experimental examples, following culturing and analysis experiments were performed using the recombinant microorganisms as prepared in this way. At this time, the wild-type strain Bacillus licheniformis GSC4071 was used as Comparative Example 1, and a cell shape thereof is shown in an upper panel of FIG. 2.

### <Experimental Example 2> 2,3-butanediol production ability of recombinant microorganism of each of Preparation Examples 1 and 2

The production ability of 2,3-butanediol and the ability to reduce the production of by-products of each of Bacillus licheniformis GSC4071 of Comparative Example 1 and the recombinant Bacillus licheniformis of each of Preparation Examples 1 and 2 were evaluated.

First, flask culturing was performed on the microorganisms as follows. The microorganisms were inoculated into a 250 ml flask containing 50 ml of a composite culture medium containing 50 g/L glucose or 50 g/L sucrose and were cultured at 45°C for 16 hours at 180 rpm. The culturing condition was set such that microaerobic condition was able to be maintained, and the culturing was carried out at an initial pH of 7.0. A sample was collected after completion of culturing of each of the wild type Bacillus licheniformis GSC4071 and the recombinant Bacillus licheniformis. The collected sample was centrifuged at 13,000 rpm for 10 minutes, and the 2,3-butanediol concentration of the supernatant was analyzed using liquid chromatography (HPLC). The viscous polymer concentration of the supernatant was measured as follows. First, the supernatant was mixed with ethanol at a 1:2 ratio, and the mixture was subjected to ethanol precipitation for 24 hours, and was centrifuged to recover the polymer therefrom. The recovered polymer was subjected to dialysis (membrane dialysis, 10 kDa) to remove low molecules therefrom, and finally, was subjected to freeze drying. Then, a dry weight thereof was measured. In order to identify polyglutamate and levan among the dried viscous polymers, the polymer was identified via NMR analysis.

As a result, when culturing the wild-type Bacillus of Comparative Example 1 in the glucose, the 2,3-butanediol production of the wild-type Bacillus of Comparative Example 1 was 16.7 g/L, the yield thereof was 0.37 g/g, and the production of the viscous polymer thereof was 3.5 g/L. When culturing the recombinant microorganism of Preparation Example 1, the 2,3-butanediol production thereof was 18.9 g/L, the yield thereof was 0.39 g/g, and the viscous polymer production thereof was 1.7 g/L.

When cultivating the wild-type Bacillus of Comparative Example 1 in the sucrose, the 2,3-butanediol production thereof was 13.8 g/L, the yield thereof was 0.26 g/g, and the viscous polymer production thereof was 14.8 g/L. When culturing the recombinant microorganism of Preparation Example 1, the 2,3-butanediol production thereof was 15.6 g/L, the yield thereof was 0.29 g/g, and the viscous polymer production thereof was 8.5 g/L. When culturing the recombinant microorganism of Preparation Example 2, the 2,3-butanediol production thereof was 14.6 g/L, the yield thereof was 0.38 g/g, and the viscous polymer production thereof was 1.8 g/L.

It was identified that when culturing the recombinant microorganism of Preparation Example 1 in the glucose culture medium, the viscous polymer production was reduced by 51% and the 2,3-butanediol production and yield increased by 13% and 5%, respectively, via deletion of the pgsBCAE gene. It may be predicted that only the sucrose culture medium affects the recombinant microorganism of Preparation Example 2. However, it was identified that when culturing the recombinant microorganism of Preparation Example 2 in the glucose culture medium, the production of the viscous polymer was reduced by 35%, compared to the recombinant microorganism of Preparation Example 1. This may because the productions of various viscous polymers that constitute a biofilm are organically related to each other, and may be due to influence of other culture medium components as added to the medium other the glucose.

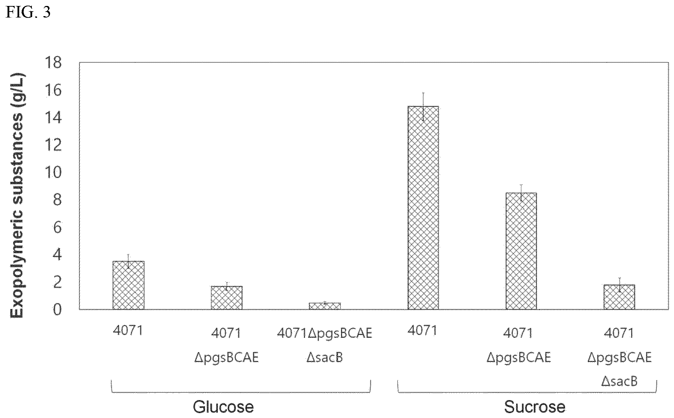

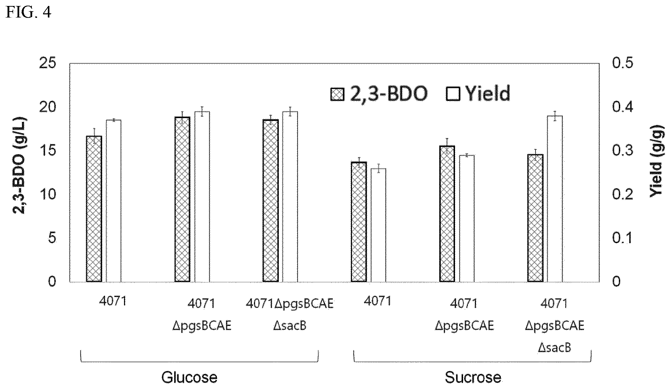

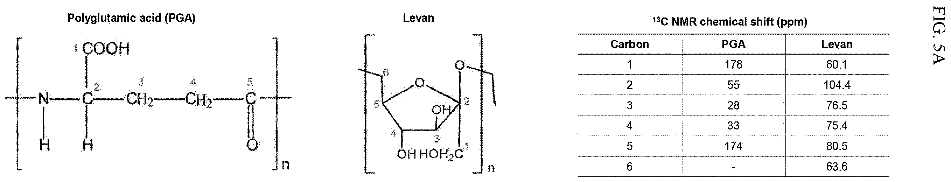

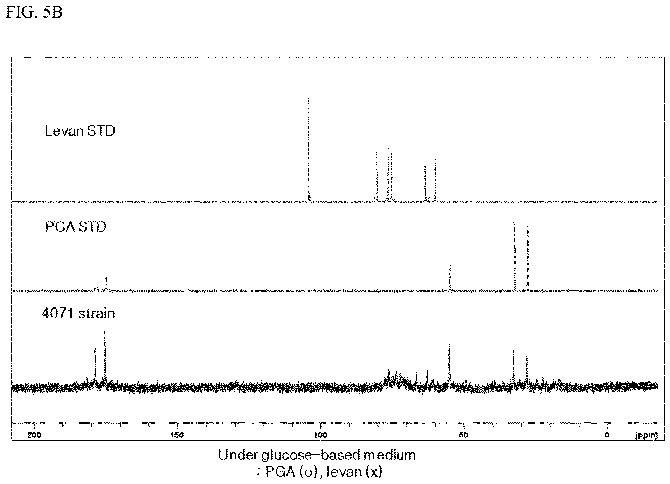

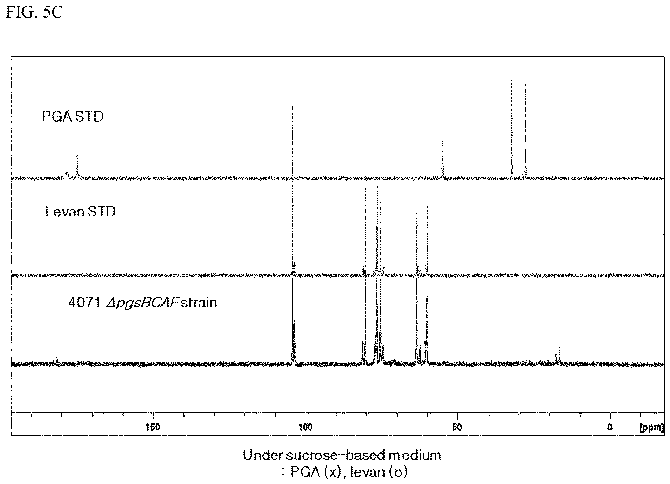

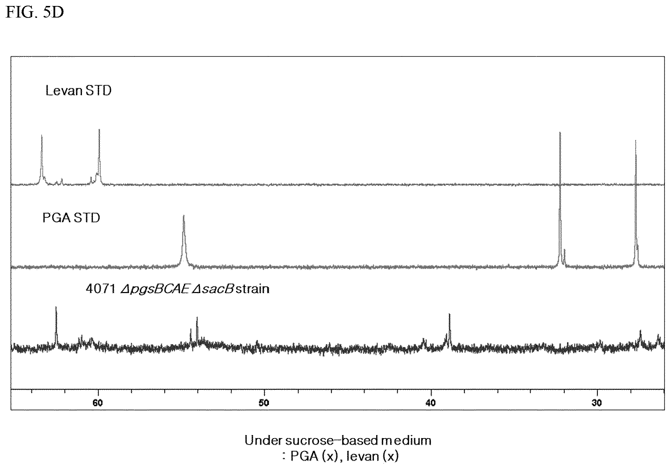

It was identified that when culturing the recombinant microorganism of Preparation Example 1 in the sucrose culture medium, the viscous polymer production was decreased by 43%, and the 2,3-butanediol production and yield were increased by 13% and 12%, respectively. Furthermore, it was identified that when culturing the recombinant microorganism of Preparation Example 2 in which the sacB gene was additionally deleted in the sucrose culture medium, the viscous polymer production was reduced by 88%, and the 2,3-butanediol production and yield were increased by 6% and 46%, respectively, compared to Comparative Example 1 as the wild-type Bacillus. As the genes related to the viscous polymer production, that is, the pgsBCAE and the sacB were sequentially deleted, the viscous polymer production gradually decreased, and the 2,3-butanediol yield was improved when the recombinant strain of each of Preparation Examples 1 and 2 was cultured in each of the glucose and sucrose culture media. It was identified based on the NMR analysis that when the recombinant microorganism of Preparation Example 1 in which the pgsBCAE was removed was cultured in the culture medium, the polyglutamate production was suppressed. It was identified based on the NMR analysis that when the recombinant microorganism of Preparation Example 2 in which the sacB was additionally removed was cultured in the culture medium, the levan production was suppressed (Table 2 and Table 3, FIG. 3, FIG. 4 and FIG. 5).

**Table 2**

| Carbon source | Glucose culture medium | | |
|---|---|---|---|
| Microorganism | Comparative Example 1 | Preparation Example 1 (decrease or increase compared to Comparative Example 1) | Preparation Example 2 (decrease or increase compared to Comparative Example 1) |
| 2,3-butanediol production (g/L) | 16.7 | 18.9 (13% ↑) | 18.6 (11% ↑) |
| 2,3-butanediol yield (g/g) | 0.37 | 0.39 (5% ↑) | 0.39 (5% ↑) |
| Viscous polymer production (g/L) | 3.5 | 1.7 (51% ↓) | 0.5 (86% ↓) |

**Table 3**

| Carbon source | Sucrose culture medium | | |
|---|---|---|---|
| Microorganism | Comparative Example 1 | Preparation Example 1 (decrease or increase compared to Comparative Example 1) | Preparation Example 2 (decrease or increase compared to Comparative Example 1) |
| 2,3-butanediol production (g/L) | 13.8 | 15.6 (13% ↑) | 14.6 (6% ↑) |
| 2,3-butanediol yield (g/g) | 0.26 | 0.29 (12% ↑) | 0.38 (46% ↑) |
| Viscous polymer production (g/L) | 14.8 | 8.5 (43% ↓) | 1.8 (88% ↓) |

### <Experimental Example 3> 2,3-butanediol production ability of recombinant microorganism of each of Preparation Examples 2 to 4

The production ability of 2,3-butanediol and a reduction of by-products of the recombinant microorganism of each of Preparation Examples 2 to 4 were evaluated.

Flask culturing was performed on each of the wild-type Bacillus licheniformis of Comparative Example 1 and the recombinant Bacillus licheniformis of each of Preparation Examples 2 to 4 as follows. First, the microorganisms were inoculated into a 250 ml flask containing 50 ml of a composite culture medium containing 50 g/L glucose and were cultured at 45°C for 16 hours at 180 rpm. The culturing condition was set such that the microaerobic condition was able to be maintained, and the culturing was carried out at an initial pH of 7.0. Samples were collected after completion of the culturing of each of the wild type and the recombinant Bacillus microorganisms, and then, the collected samples were centrifuged at 13,000 rpm for 10 minutes. Then, the concentrations of the 2,3-butanediol, the lactate, and the glycerol in the supernatant were analyzed using liquid chromatography (HPLC).

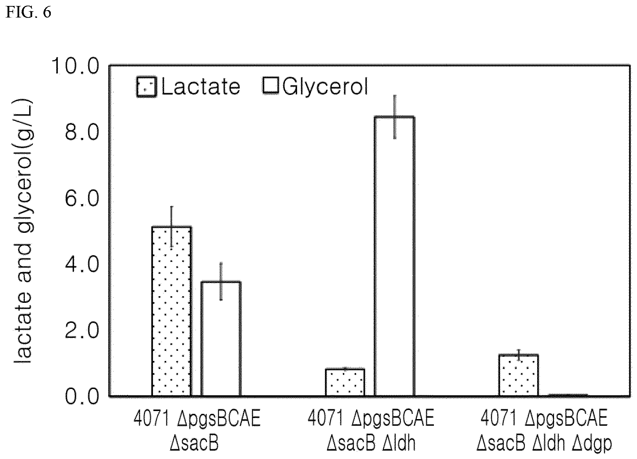

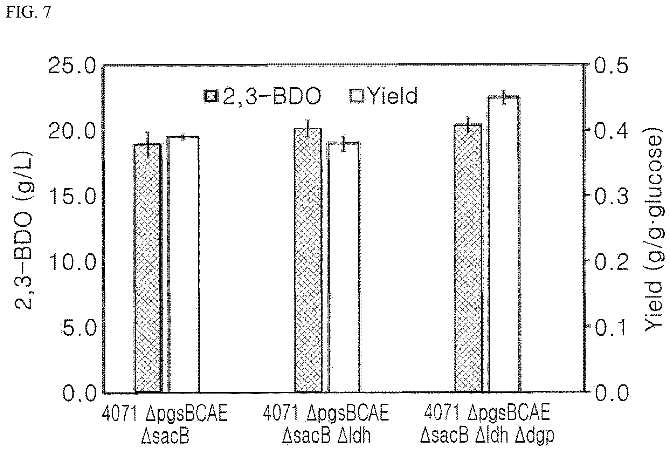

As a result, when culturing the wild-type Bacillus licheniformis of Comparative Example 1, the 2,3-butanediol production was 16.7 g/L, the yield thereof was 0.37 g/g, the lactate production was 5.25 g/L, and the glycerol production was 4.97 g/L. When culturing the recombinant microorganism of Preparation Example 2, the 2,3-butanediol production was 19.0 g/L, the yield thereof was 0.39 g/g, the lactate production was 5.13 g/L, and the glycerol production was 3.47 g/L. No significant reduction in each of the lactate and glycerol productions via removal of each of the polyglutamate and the levan was observed. When culturing the recombinant microorganism of Preparation Example 3, the 2,3-butanediol production was 20.14 g/L, the yield thereof was 0.39 g/g, the lactate production was 0.832 g/L, and the glycerol production was 8.45 g/L. When culturing the recombinant microorganism of Preparation Example 4, the 2,3-butanediol production was 20.42 g/L, the yield thereof was 0.45 g/g, the lactate production was 1.25 g/L, and the glycerol production was 0.04 g/L. The recombinant microorganism of Preparation Example 3 had the increased 2,3-butanediol production and the lactate production decreased by 84%, compared to the recombinant microorganism of Preparation Example 2, but had the glycerol production increased by 144%, compared to the recombinant microorganism of Preparation Example 2. Thus, the recombinant microorganism of Preparation Example 3 had insignificant effect in improving the 2,3-butanediol yield. The recombinant microorganism of Preparation Example 4 had the 2,3-butanediol yield increased by about 13% compared to the recombinant microorganism of Preparation Example 2, and had the lactate production reduced by 75%, compared thereto, and produced substantially no glycerol. Thus, it may be identified that each of the lactate and glycerol productions was significantly reduced by removing each of the ldh and dgp genes. When the both genes were deleted, not only the 2,3-butanediol production but also the yield thereof were significantly improved. (Table 4, FIG. 6 and FIG. 7).

**Table 4**

| Carbon source | Glucose culture medium | | | |
|---|---|---|---|---|
| Microorganism | Comparative Example 1 | Preparation Example 2 | Preparation Example 3 (decrease or increase compared to Preparation Example 2) | Preparation Example 4 (decrease or increase compared to Preparation Example 2) |
| 2,3-butanediol production (g/L) | 16.7 | 19 | 20.14 (6% ↑) | 20.42 (7.5% ↑) |
| 2,3-butanediol yield (g/g) | 0.37 | 0.39 | 0.39 | 0.45 (13% ↑) |
| Lactate production (g/L) | 5.25 | 5.13 | 0.832 (84% ↓) | 1.25 (76% ↓) |
| Glycerol production (g/L) | 4.97 | 3.47 | 8.45 (144% ↑) | 0.04 (negligible ↓) |

### <Experimental Example 4>

Fed-batch fermentation performance was evaluated under glucose and sucrose culture medium conditions using the recombinant microorganism of Preparation Example 4.

The microorganism fermentation experiment was performed as follows. First, the microorganism was inoculated into a 1L flask containing 300 ml of a composite culture medium and was cultured at 45°C for 16 hours at 180 rpm. The cultivated microorganisms were inoculated into a fermenter with a capacity of 7.5 L so that a volume of a final culturing product was 3 L. The initial conditions were pH 7.0, temperature 45°C, a stirring speed 550 rpm, and oxygen 1 wm. The initial concentration of glucose or sucrose was set to 100 g/L. During the culturing, when an amount of acetoin exceeded 10g/L, a second-step fermentation was performed in which the stirring speed was lowered to 350 rpm. When the sugar concentration in the culturing solution fell below 20 g/L, 150 g of the glucose or sucrose powder was additionally fed thereto. Samples were collected after the culturing of the recombinant Bacillus, and cell growth was measured as an OD600 value using a spectrophotometer. The collected samples were centrifuged at 13,000 rpm for 10 minutes, and then the concentrations of the glucose, the sucrose, the 2,3-butanediol, and the organic metabolites in the supernatant were analyzed using liquid chromatography (HPLC).

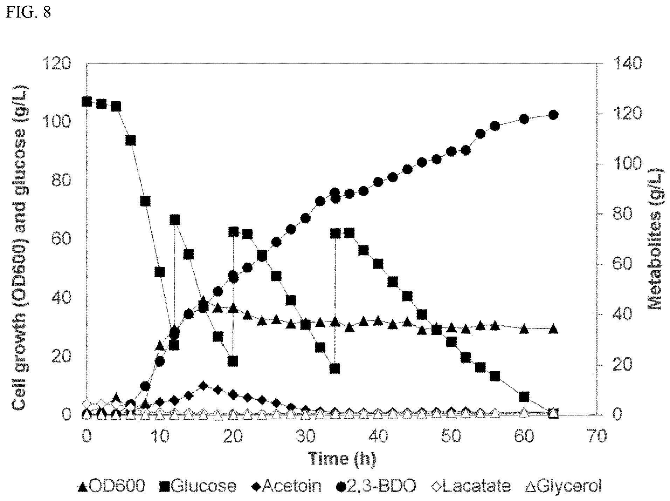

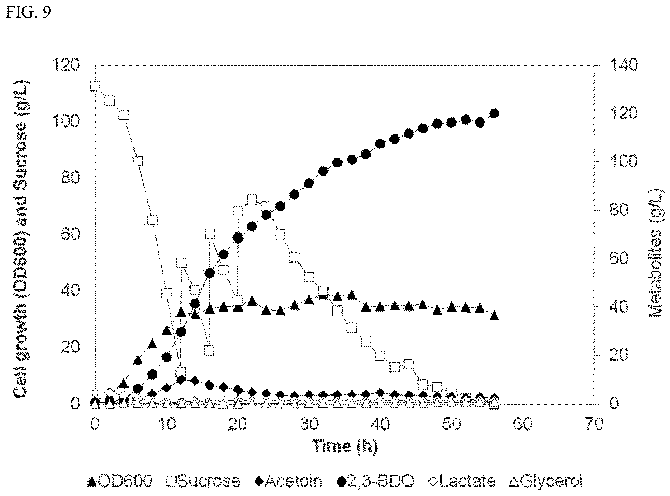

As a result, when the fed-batch fermentation was performed in the glucose, the 2,3-butanediol production was 119.6 g/L, the yield thereof was 0.42 g/g, and the productivity thereof was 1.87 g/L/h. Similarly, when the fed-batch fermentation was performed in the sucrose, the 2,3-butanediol production was 120.1 g/L, the yield thereof was 0.42 g/g, and the productivity thereof was 2.14 g/L/h. The viscous polymers and organic metabolites were produced only at trace levels that did not affect the 2,3-butanediol fermentation. It was identified that via the fed-batch fermentation, the recombinant microorganisms are capable of producing industrial-level high concentrations of 2,3-butanediol from both glucose and sucrose culture mediums (Table 5, FIG. 8 and FIG. 9).

**Table 5**

| Carbon source | Glucose | Sucrose |
|---|---|---|
| 2,3-butanediol production (g/L) | 119.6 | 120.1 |
| 2,3-butanediol yield (g/g) | 0.42 | 0.42 |
| 2,3-butanediol productivity (g/L/h) | 1.87 | 2.14 |

### Industrial applicability

The present disclosure provides the microorganism with the 2,3-butanediol biosynthetic pathway, wherein the microorganism includes the recombinant microorganism for producing 2,3-butanediol, wherein at least one selected from a group consisting of the path for converting glutamate to polyglutamate, the path for converting sucrose to levan, the path for converting pyruvate to lactate and the path for converting glycerophosphate to glycerol is inhibited. Moreover, the present disclosure provides the production method of 2,3-butanediol using the recombinant microorganism for producing the 2,3-butanediol as described above.

### Sequence Listing Free text

SEQ ID NO: 1: A base sequence of pgsBCAE.
SEQ ID NO: 2: A base sequence of a homologous region 1 of pgsBCAE.
SEQ ID NO: 3: A base sequence of a primer for amplifying the homologous region 1 of pgsBCAE via PCR.
SEQ ID NO: 4: A base sequence of a primer for amplifying the homologous region 1 of pgsBCAE via PCR.
SEQ ID NO: 5: Abase sequence of a homologous region 2 of pgsBCAE.
SEQ ID NO: 6: A base sequence of a primer for amplifying the homologous region 2 of pgsBCAE via PCR.
SEQ ID NO: 7: A base sequence of a primer for amplifying the homologous region 2 of pgsBCAE via PCR.
SEQ ID NO: 8: A base sequence of a DNA fragment including the homologous regions 1 and 2 of pgsBCAE.
SEQ ID NO: 9: Abase sequence of sacB.
SEQ ID NO: 10: Abase sequence of a homologous region 1 of sacB.
SEQ ID NO: 11: A base sequence of a primer for amplifying the homologous region 1 of sacB via PCR.
SEQ ID NO: 12: A base sequence of a primer for amplifying the homologous region 1 of sacB via PCR.
SEQ ID NO: 13: A base sequence of a homologous region 2 of sacB.
SEQ ID NO: 14: Abase sequence of a primer for amplifying the homologous region 2 of sacB via PCR.
SEQ ID NO: 15: A base sequence of a primer for amplifying the homologous region 2 of sacB via PCR.
SEQ ID NO: 16: A base sequence of a DNA fragment including the homologous regions 1 and 2 of sacB.
SEQ ID NO: 17: A base sequence of ldh.
SEQ ID NO: 18: Abase sequence of a homologous region 1 of ldh.
SEQ ID NO: 19: Abase sequence of a primer for amplifying the homologous region 1 of ldh via PCR.
SEQ ID NO: 20: A base sequence of a primer for amplifying the homologous region 1 of ldh via PCR.
SEQ ID NO: 21: A base sequence of a homologous region 2 of ldh.
SEQ ID NO: 22: A base sequence of a primer for amplifying the homologous region 2 of ldh via PCR.
SEQ ID NO: 23: A base sequence of a primer for amplifying the homologous region 2 of ldh via PCR.
SEQ ID NO: 24: A base sequence of a DNA fragment containing the homologous regions 1 and 2 of ldh.
SEQ ID NO: 25: A base sequence of dgp.
SEQ ID NO: 26: A base sequence of a homologous region 1 of dgp.
SEQ ID NO: 27: Abase sequence of a primer for amplifying the homologous region 1 of dgp via PCR
SEQ ID NO: 28: A base sequence of a primer for amplifying the homologous region 1 of dgp via PCR.
SEQ ID NO: 29: Abase sequence of a homologous region 2 of dgp.
SEQ ID NO: 30: A base sequence of a primer for amplifying the homologous region 2 of dgp via PCR.
SEQ ID NO: 31: Abase sequence of a primer for amplifying the homologous region 2 of dgp via PCR.
SEQ ID NO: 32: A base sequence of a DNA fragment including the homologous regions 1 and 2 of dgp.

Name of depository institution: Korea Research Institute of Bioscience and Biotechnology
Accession number: KCTC14485BP
Accession date: 20210305

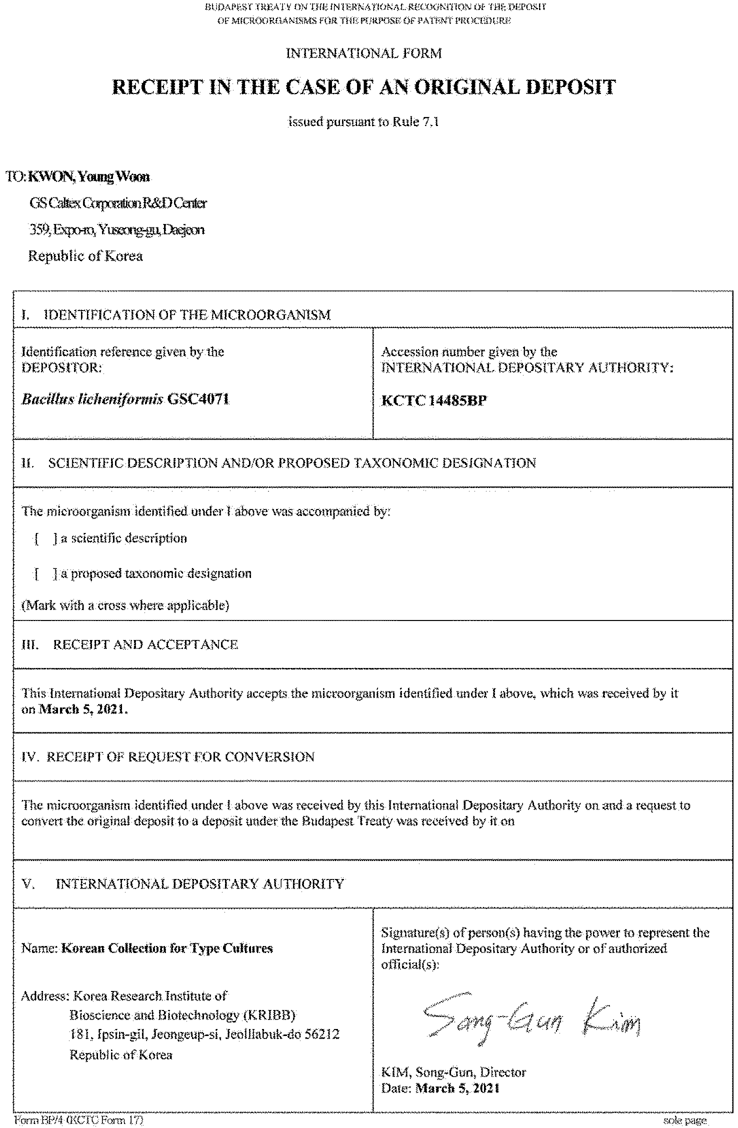
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KWON, Young Woon
GS Caltex Corporation R&D Center
359, Expo-ro, Yuseong-gu, Daejeon
Republic of Korea

I. IDENTIFICATION OF THE MICROORGANISM
Identification reference given by the
DEPOSITOR:

Bacillus licheniformis GSC4071 | Accession number given by the
INTERNATIONAL DEPOSITARY AUTHORITY:

KCTC 14485BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on March 5, 2021.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: March 5, 2021

Form BP/4 (KCTC Form 17) sole page

## Claims

1. A recombinant microorganism for producing 2,3-butanediol, wherein the microorganism has a 2,3-butanediol biosynthetic pathway, wherein at least one selected from a group consisting of a polyglutamate biosynthetic pathway, a levan biosynthetic pathway, a lactate biosynthetic pathway, and a glycerol biosynthetic pathway is inhibited.

2. The recombinant microorganism for producing 2,3-butanediol of claim 1, wherein the polyglutamate biosynthetic pathway is a path for converting glutamate to polyglutamate.

3. The recombinant microorganism for producing 2,3-butanediol of claim 1, wherein the levan biosynthetic pathway is a path for converting sucrose to levan.

4. The recombinant microorganism for producing 2,3-butanediol of claim 1, wherein the lactate biosynthetic pathway is a path for converting pyruvate to lactate.

5. The recombinant microorganism for producing 2,3-butanediol of claim 1, wherein the glycerol biosynthetic pathway is a path for converting glycerophosphate to glycerol.

6. The recombinant microorganism for producing 2,3-butanediol of claim 1, wherein a production of a viscous polymer, lactate or glycerol in cultivating the recombinant microorganism is lower than a production of the viscous polymer, the lactate or the glycerol in cultivating a wild type microorganism.

7. The recombinant microorganism for producing 2,3-butanediol of claim 1, wherein the recombinant microorganism has a production ability of 2,3-butanediol higher than a production ability of 2,3-butanediol of a wild-type microorganism.

8. The recombinant microorganism for producing 2,3-butanediol of claim 1, wherein a path for converting glutamate to polyglutamate is inhibited, while a path for converting sucrose to levan, a path for converting pyruvate to lactate, and a path for converting glycerophosphate to glycerol are not inhibited.

9. The recombinant microorganism for producing 2,3-butanediol of claim 1, wherein at least one selected from a group consisting of a path for converting glutamate to polyglutamate, a path for converting sucrose to levan, a path for converting pyruvate to lactate, and a path for converting glycerophosphate to glycerol is inhibited.

10. The recombinant microorganism for producing 2,3-butanediol of claim 1, wherein a path for converting glutamate to polyglutamate and a path for converting sucrose to levan are inhibited, while a path for converting pyruvate to lactate and a path for converting glycerophosphate to glycerol are not inhibited.

11. The recombinant microorganism for producing 2,3-butanediol of claim 1, wherein a path for converting glutamate to polyglutamate, a path for converting sucrose to levan, and a path for converting pyruvate to lactate are inhibited, while a path for converting glycerophosphate to glycerol is not inhibited.

12. The recombinant microorganism for producing 2,3-butanediol of claim 1, wherein a path for converting glutamate to polyglutamate, a path for converting sucrose to levan, a path for converting pyruvate to lactate, and a path for converting glycerophosphate to glycerol are inhibited.

13. A method for producing 2,3-butanediol, the method comprising:
culturing the recombinant microorganism for producing 2,3-butanediol of one of claims 1 to 12; and
recovering 2,3-butanediol from the culturing product.
